# EUROPEAN PATENT APPLICATION

(11) **EP 1 843 268 A1**
(43) Date of publication of application: **10.10.2007**
(21) Application number: 06007269.1
(22) Date of filing: 06.04.2006
(51) Int. Cl.: G06F 19/00

(54) **Transfer of treatment planning information using standard image transfer protocols**

(71) Applicant: BrainLAB AG, 85551 Kirchheim/Heimstetten (DE)
(72) Inventor: Hartlep, Andreas, Dr., 83629 Weyarn (DE); Frielinghaus, Nils, 85551 Heimstetten (DE)
(74) Representative: Schwabe - Sandmair - Marx

(57) **Abstract**

The invention concerns a method for creating, generating, saving or exporting medical data including image data (300) which conforms to an external standard wherein additional data (330) not being as additional data part of the external standard, is added to the image data (300) by rendering the additional data (330), as e.g. treatment planning information, into the image data (300) to create new data comprising at least part of the original image data (300) and at least a part of the additional data (330), wherein the new data conforms to the external standard and a medical image generating device (100) comprising an imaging device (101) and a processor (130) connected to the imaging device (101) obtaining image data conforming to an external standard from the imaging device (101) and rendering additional image data (330) into the image data (300) from the imaging device (101) to generate new data including at least part of the additional image data (330), the new data conforming to the external standard.

## Description

The present invention relates to a method and a device for generating and / or exporting and /or saving data, especially medical image data, conforming to an external standard, as e.g. DICOM, including additional image information or image data.

Because medical imaging equipment typically must interoperate with other medical devices, it is common for CT, MR, PET or SPECT scanners or other medical imaging devices, displays and software to be interoperable and to exchange data based on a common standard or protocol.

An example for such a protocol is the DICOM (digital imaging and communication in medicine) standard published by the National Electrical Manufacturers Association and available at http://medical.nema.org/dicom/2000.html. The DICOM standard includes syntax and semantics of commands and associated information, which can be exchanged by devices, particularly medical imaging equipment, using the protocol. By using an interoperability standard, the exchange of digital information between medical imaging equipment can be facilitated and medical image data can be easily shared and used among compliant devices, such as imaging systems and workstations. Preferably, interfaces are based on a standard such as the DICOM standard.

A typical use of DICOM is with CT or MR images. Because the image data is typically processed, post-scan converted data, once the MR or CT images are stored, none of the post-processing capabilities normally available on the MR or CT system, such as gray-scale maps, edge enhancement, and video filters, are available to enhance the CT or MR image. This provides the benefit of ensuring that the archived image reproduces as closely as possible what the clinician who stored the image was viewing at the time the image was archived.

Figure 4 shows in principle a data set representing medical image data according to the DICOM standard including image data 300 and, as header, public and private additional data 320 and 310. The standardized public data 320 includes information concerning the interpretation or "meaning" of the image data 300.

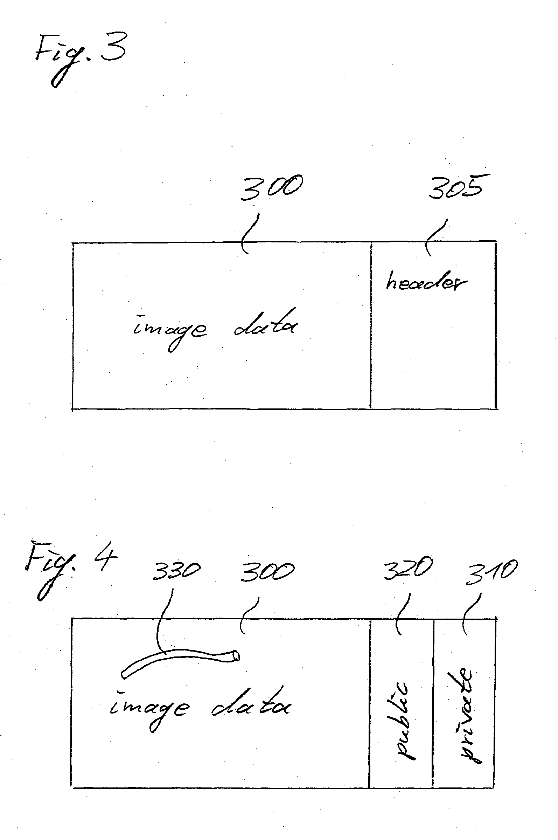

If image data not conforming to the DICOM standard being specific for the applicant's devices and software and being shown in principle in Fig. 3, is used, additional information can be added in the header 305. This additional information can be treatment data defining e.g. trajectories or surgical pathways for planning the treatment of the tissue or any other kind of data.

A clinician would probably also like to add further information, as e.g. further image data, for planning a treatment, to the DICOM data, which further image data might be helpful for the further use of the DICOM data e.g. for surgical planning.

However, the DICOM standard does not allow adding additional image data like treatment data e.g. trajectory information.

US 2005/0074157 A1 discloses a method for displaying or manipulating medical image data, wherein a file in compliance with a medical image standard is provided to a medical image viewer, the medical image standard specifies a first field for data not in compliance with the medical image standard and a second field for data in compliance with the medical image standard, wherein the first field of the file comprises medical image data and the second field of the file comprises information that can be used to obtain software to at least display or manipulate the medical image data. Using the obtained software, the medical image data is displayed or manipulated.

It is an object of the present invention to provide a method and a device for generating, saving or exporting data conforming to an external standard while adding additional information.

This object is solved by the features of the independent claims. Preferred embodiments are defined in the dependent claims.

According to the method for creating, generating or exporting medical data including treatment planning data, such as surgical planning data, which conforms to an external standard, as e.g. the DICOM standard, an additional information, which can be additional image data, not being as additional information part of the external standard, is added to the image data conforming to the external standard by rendering the additional data, as e.g. treatment planning information, directly into the image data conforming to the external standard to create new data comprising at least part of the original image data conforming to the external standard and at least a part of the additional data, wherein the thus new generated data conforms to the external standard and can be exported to be used by other devices.

Thus, using the above method, data can be added to the DICOM standard, which does not regularly allow representing medical treatment planning information, like e.g. surgical access paths or trajectories. According to the invention it is now possible that surgical planning data that includes trajectory information conformal to an external standard can be exported without changing the external standard or using non-public and thus not general supported header entries.

In other words, the image data 300 itself of the DICOM data is modified by altering or replacing parts of the image data 300, as e.g. specific pixels, to add or draw additional image data 330, representing e.g. trajectories, directly into the image data 300 without modifying the header 310, 320 of the DICOM data.

Exporting data conformal to a standard (e.g. DICOM) that is supported by a variety of systems that contains planning information 330 that directly is rendered into the image data 300 provides the following advantages: Internal data of a medical system can be made accessible to "foreign systems" without disclosing internal standards, even if the common standard (e.g. DICOM) does not allow to add graphic objects. The external standard need not to be changed or adapted and thus, full compatibility is provided.

The invention enables thus the transfer of medical data, e.g. imaging or treatment planning data like trajectories, using standard image transfer protocols like DICOM. This transfer can be performed by loading image data that is in spatial relation to the treatment planning information and rendering the treatment planning information into the images, thus replacing, changing or manipulating the original image data at those points where the treatment planning information is represented or located such that the treatment planning information is visible in the data. The changes and manipulations might include changes of the (image) information and might include additions to the (image) information, e.g. objects that might have been rendered into the data set. The resulting images carrying the medical treatment planning information can be transferred using a standard image transfer protocol like DICOM and can be imported into any kind of application that is compatible with this protocol.

One embodiment is the display of patient data using a system-internal imaging data standard, and the use of this image data set to plan trajectories of instruments or catheters for surgical treatments. To make this plans (image data and the trajectory) accessible to systems that are not supporting the used data standard, all objects (e.g. trajectories) are rendered directly into the image data set and this newly created data set is stored conformal to a standard (e.g. DICOM) that is supported by the other imaging viewing or processing system.

The embodiments described below relate to methods and systems for displaying and/or manipulating medical image data. In one embodiment, a medical image viewer in compliance with a medical image standard is provided, and a file in compliance with the medical image standard is provided to the medical image viewer. The medical image standard specifies in addition to image data a first field for data not being standardized by the medical image standard, see field "private" in FIG. 4, and a second field for data being standardized by the medical image standard, see the field "public" in FIG. 4.

The embodiments will now be described with reference to the attached drawings.

FIG. 1 is a block diagram of a medical diagnostic CT imaging system of an embodiment.

FIG. 2 is a block diagram of a medical image viewer of an embodiment.

FIG. 3 is an illustration of a user specific medical image standard of an embodiment.

FIG. 4 is an illustration of the DICOM standard.

The embodiments described below relate generally to diagnostic medical images. Although any type of medical image can be used, these embodiments will be illustrated in conjunction with CT images. As noted in more detail below, other types of medical images can be used, and the following claims should not be limited to CT images unless explicitly recited therein.

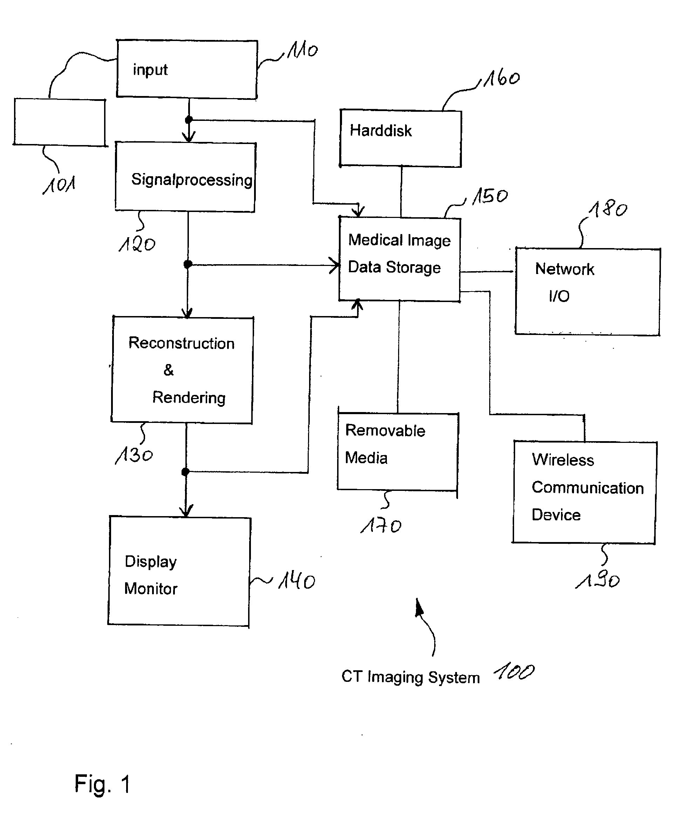

FIG. 1 is a block diagram of a CT imaging system 100 comprising a CT scan data input port 110 being connected to a CT scanner 101, a signal processing section 120, a reconstruction and rendering section 130, and a display monitor 140. The CT system 100 also comprises a medical image data storage section 150, which can capture image data at one or more locations along the image path, a hard disk 160, removable media 170 (e.g., a CD, a DVD, etc.), a network I/O port 180, and a wireless communication device 190.

During a CT examination, the CT scanner 101 produces image data referred to as "CT data" and sends this image data via the input port 110 to the signal processing section 120. The signal processing section 120 is used to evaluate the CT data and transmit it e.g. as DICOM data directly to storage section 150 or as data to be modified according to the invention to the reconstruction and rendering section 130 which can be provide rendered data to the display 140 and storage section 150.

The medical image data storage section 150 captures image data, which is DICOM compliant and can consist of 2D images, 3D data or rendered 2D or 3D data. The image data outputted by the signal processing section 120 could be 2D image data or 3D image data before scan conversion, rendering or reconstruction. This image data can be readable by DICOM-compliant devices such as DICOM workstations. The captured image data can also be stored in the hard disk 160 and/or removable media 170. The captured image data can also be exported from the CT system 100 via the network I/O 180 (e.g., across an intranet or the Internet) or via the wireless communication device 190.

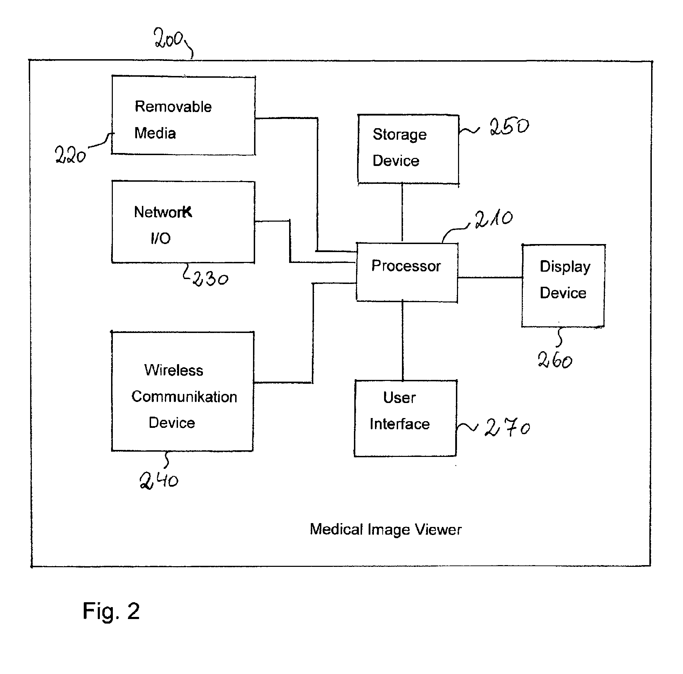

The CT system 100 operates in compliance with a medical image standard and sends captured medical image data to another device operating in compliance with the medical image standard. Although any medical image standard now existing or developed in the future can be used, the DICOM standard will be used to illustrate this embodiment. In operation, the medical image data storage section 150 packages the medical image data in a file that is sent to a medical image viewer as shown in FIG. 2 via removable media 170, the network I/O 180, or the wireless communication device 190. As used herein, the term "medical image viewer" broadly refers to any device that can be used to view and/or manipulate medical image data. Examples of medical image viewers include, but are not limited to, dedicated workstation (i.e., image review stations), general-purpose computers, personal digital assistants, cell phones, and set-top boxes. A medical image viewer can also be a medical imaging system (e.g., a CT system) different from the one used to generate the medical image data.

FIG. 2 is a block diagram of a medical image viewer 200 of an embodiment. As shown in FIG. 2, the medical image viewer 200 comprises a processor 210 in communication with removable media 220, a network I/O 230, and a wireless communication device 240 that interfaces with the CT system's removable media 170, network I/O 180, and the wireless communication device 190, respectively. The medical image viewer 200 also comprises a storage device 250 that can store the transferred medical image data file (and/or computer-readable program code executable by the processor 210), a display device 260, and a user interface 270.

Because the CT system 100 and medical image viewer 200 operate in compliance with the same medical image standard, medical image data that is in compliance with the medical image standard can be viewed by the medical image viewer 200 (i.e., the processor 210 of the medical image viewer 200 runs medical-image-standard-compliant viewing software). With this embodiment, the medical image viewer 200 can also display and/or manipulate medical image data that is not in compliance with the medical image standard (e.g. pre-scan converted data, pre-reconstruction data, and a three-dimensional data set). This embodiment provides the feature of incorporating additional image data and/or manipulating medical images into the image data file sent to the medical image viewer 200. This will be discussed in more detail with reference to FIG. 4.

As shown in FIG. 4, the medical image standard specifies that a file associated with medical image data has in addition to the medical image data 300 a first field 310 and a second field 320 (the medical image standard can also specify additional fields not shown in FIG. 4 for simplicity). The first field 310 is for data that is not standardized in compliance with the medical image standard, and the second field 320 is for data that is standardized in compliance with the medical image standard. In the DICOM medical image standard, the first field 310 is the DICOM private attribute, and the second field 320 is the DICOM standard or public attribute.

One example of an additional image data is data showing trajectories 330 for a planned insertion of a catheter into the tissue shown in the image data 300. This additional image data 330 is rendered directly into the original image data 300, e.g. by modifying or replacing the pixels at the location of the planned trajectories without modifying any of the private or public fields 310 or 320. The advantage of this approach is that it does not require any new special capability from the DICOM workstation.

It is noted that this modification of the original image data 300 can be made either in the CT system 100 shown in FIG. 1, in the image viewer 200 shown in FIG. 2 or at any other location or decive.

In summary, the embodiment described herein can be used to allow a user to store medical image data 300 that is in an industry standard format (such as DICOM) including additional information 330 that is not part of the original medical image.

## Claims

1. Method for creating, generating, saving and / or exporting medical data including image data (300) which conforms to an external standard wherein additional data (330), not being as additional data part of the external standard, is added to the image data (300) by rendering the additional data (330), as e.g. treatment planning information, into the image data (300) to create new data comprising at least part of the original image data (300) and at least a part of the additional data (330), wherein the new data is conformal to the external standard.

2. Method according to claim 1, wherein the external standard is the DICOM standard.

3. Method according to one of the previous claims, wherein the additional data (330) is treatment planning information such as trajectory information, surgical pathways, labels or others.

4. Method according to one of the previous claims, wherein rendering the additional data (330) into the image data (300) conforming to the external standard comprises at least one of changing the image data and adding the additional data.

5. Medical image generating device (100) comprising an imaging device (101) and a processor (130) connected to the imaging device (101) obtaining image data conforming to an external standard from the imaging device (101) and rendering additional image data (330) into the image data (300) from the imaging device (101) to generate new data including at least part of the additional image data (330), the new data conforming to the external standard.

6. Device according to claim 5, wherein the external standard is the DICOM standard.

7. Device according to claim 5, wherein the additional data (330) is treatment planning information such as trajectory information, surgical pathways, labels or others.

8. Device according to claim 5, wherein rendering the additional data (330) into the image data (300) conforming to the external standard comprises at least one of changing the image data and adding the additional data.

9. Computer program which, when loaded or running on a computer, performs the method of one claims 1 to 4.

10. Program storage medium or computer program product comprising the program of the previous claim.

11. Medical imaging generating device according to the previous claim, wherein the imaging device (101) is a PACS system or a CT scanner, MR scanner, PET scanner, SPECT scanner or any other medical data acquisition device.

12. DICOM image data including additional image data not conforming to the DICOM standard.
